# EUROPÄISCHE PATENTSCHRIFT

(11) **EP 0 634 361 B1**
(45) Veröffentlichungstag und Bekanntmachung des Hinweises auf die Patenterteilung: **23.04.1997**
(21) Anmeldenummer: 94110464.8
(22) Anmeldetag: 05.07.1994
(51) Int. Cl.: C01B 39/36, C01B 39/12, C01B 39/08, B01J 29/40, B01J 29/86, B01J 29/89, B01J 20/18

(54) **Hohlkugelartig agglomerierte Pentasilzeolithe**
Hollow spheroidal agglomerated pentasil zeolites
Zeolites pentasil agglomérées sphéroidales creuses

(30) Priorität: 15.07.1993 DE 4323774
(43) Veröffentlichungstag der Anmeldung: 18.01.1995
(73) Patentinhaber: BASF Aktiengesellschaft, 67063 Ludwigshafen (DE)
(72) Erfinder: Mueller, Ulrich, Dr., D-67434 Neustadt (DE); Reich, Axel, Dr., D-55288 Schornsheim (DE); Unger, Klaus, Prof. Dr., D-64342 Seeheim-Jugenheim (DE)

(56) Entgegenhaltungen:
- EP-A- 0 007 081
- EP-A- 0 034 727
- EP-A- 0 041 621
- EP-A- 0 065 401
- EP-A- 0 072 054
- EP-A- 0 219 804

## Beschreibung

Die vorliegende Erfindung betrifft hohlkugelartige agglomerierte Pentasilzeolithe mit einer Partikelgröße von 5 bis 200 micron, deren Herstellung durch Behandlung einer Mischung aus einem wäßrigen Alkylen-di-, -tri- und/oder -tetramin, Siliciumdioxid und einem Aluminium- Bor- und/oder Titansalz bei Temperaturen von 20 bis 100°C erfolgt sowie deren Verwendung als Katalysatoren oder Adsorbentien.

Zeolithe sind bekanntermaßen kristalline Alumosilikate mit geordneten Kanal- und Käfigstrukturen, deren Porenöffnungen im Bereich von Mikroporen kleiner 0,9 nm liegen. Das Netzwerk solcher Zeolithe ist aufgebaut aus SiO₄ und AlO₄-Tetraedern, die über gemeinsame Sauerstoffbrücken verbunden sind. Eine Übersicht der bekannten Strukturen findet sich beispielsweise bei W.M. Meier und D.H. Olson, "Atlas of Zeolite Structure Types", 2. Auflage, Butterworths, London 1987.

Zum Ausgleich der negativen Elektrovalenz die durch den Einbau von Al(III) in das Si(IV)-Silikatgitter entsteht, findet man bei Zeolithen austauschfähige Kationen. Ersetzt man diese Kationen gegen Protonen, beispielsweise durch einen Ionenaustausch, so erhält man die entsprechend aziden Festkörper mit Zeolithstruktur, die sogenannte H-Form. Die gebräuchlichsten synthetisch herstellbaren Typen wie Zeolith-A, X und Y finden als Molekularsiebe, Ionenaustauscher und Katalysatoren technische Anwendungen. Für die Verwendung als Katalysatoren sind aufgrund ihrer thermischen Stabilität und ihrer Azidität insbesondere siliziumreiche Pentasilzeolithe mit von Interesse. Bei diesen Materialien kann unter Erhalt des Strukturtyps das im Kristallgitter eingebaute Aluminium durch Eisen, Gallium oder Bor ersetzt werden, wodurch man Pentasilzeolithe mit einer abgestuften Azidität erhält (Post, Huizinga, Emeis, Nanne, Stork in "Zeolites as Catalysts, Sorbents and Detergent Builders" und Karge, Weitkamp (Eds.); Elsevier (Amsterdam) 1989, Seite 365 bis 375)

Solche aciden Pentasilzeolithe können für heterogenkatalytische Umsetzungen organischer Moleküle eingesetzt werden. Bei der Herstellung organischer Zwischenprodukte finden Pentasilzeolithe Verwendung bei der Herstellung von 3-Acylpyridin aus Acrolein, Ketonen in Acetalform und Ammoniak (EP-A-318 845), bei der Synthese von Aminen aus Olefinen und Ammoniak (DE-A-36 34 247), bei der Isomerisierung von Epoxiden (DE-A-35 46 372), sowie bei der Vinylethersynthese aus Acetalen (DE-A-37 22 891) um nur einige Beispiele zu nennen.

Bei der Herstellung von Pentasilzeolithen kennt man nach dem Stand der Technik die Möglichkeiten, wäßrige alkalische Mischungen aus einer Silicaquelle, einer Metalloxidquelle, Natriumoxid und einer organischen Schablonenverbindung wie beispielsweise Tetra-n-propylammoniumsalzen hydrothermai unter autogenem Druck umzusetzen. Solche Verfahren werden beispielsweise in EP-A-68 796 beschrieben. Die Verwendung von Piperidin oder Hexamethylenimin als organische Schablonenverbindung ist aus EP-A-293 032 bekannt und in DE-A-28 30 787 bzw. in EP-A-7 081 ist gezeigt, daß man in einer alkalifreien Synthesemischung mit Hexa-methylendiamin ebenfalls Metallsilikatzeolithe erhält.

Aus technischen Kristallisationen erhält man Zeolithe in Form feinteiliger Pulver mit Teilchengrößen von 0,5 bis 3 micron. Insbesondere die Abtrennung (Zentrifugation, Filtracion, waschung), der Umgang und die Verarbeitung solcher Materialien zu Katalysatoren oder Adsorbentien ist hinsichtlich Arbeitsschutz (Feinstaub) zunehmend problematisch, zeit- und damir kostenintensiv.

Der vorliegenden Erfindung lag daher die Aufgabe zugrunde, den zuvor genannten Nachteilen abzuhelfen, und Pentasilzeolithe in einer leicht handhabbaren Form verfügbar zu machen.

Demgemäß wurden neue hohlkugelartige agglomerierte Pentasilzeolithe mit einer Partikelgröße von 5 bis 200 micron und ein Verfahren zur Herstellung dieser hohlkugelartig agglomerierten Pentasilzeolithen gefunden, welches dadurch gekennzeichnet ist, daß man eine Mischung aus einem wäßrigen Alkylendi-, -tri- und/oder -tetramin, Siliciumdioxid und einem Aluminium-, Bor- und/oder Titansalz bei Temperaturen von 20 bis 100°C behandelt, anschließende bei Temperaturen von 110 bis 200°C und Drücken von 0,01 bis 50 bar kristallisiert und bei Temperaturen von 400 bis 650°C calciniert sowie deren Verwendung als Katalysatoren und/oder Adsorbentien.

Die erfindungsgemäßen hohlkugelartig agglomerierten Pentasilzeolithe lassen sich wie folgt herstellen:

Man kann eine wäßrige Alkylendi-, -tri- und/oder -tetraminlösung als 20 bis 75 gew.-%ige, bevorzugt 35 bis 60 gew.-%ige, besonders bevorzugt 50 gew.-%ige Lösung z.B. in einem Druckbehälter vorlegen und die SiO₂-Quelle, beispielsweise Siliciumdioxid, Kieselgel, pyrogenes Silica besonders bevorzugt pyrogen hergestelltes Silica, unter Rühren z.B. bei Temperaturen von 10 bis 40°C, in der Regel bei Raumtemperatur 18 bis 25°C einbringen. Als Rührwerke eignen sich beispielsweise Anker-Rührwerke. Die Rührgeschwindigkeit beträgt in der Regel 20 bis 500 U/min, bevorzugt 40 bis 300 U/min, besonders bevorzugt 50 bis 150 U/min.

In einem separaten Mischgefäß kann man das Aluminium-, Bor- und/oder Titansalz, bevorzugt Aluminium- und/oder Borsalz, besonders bevorzugt Aluminiumsalz wie Halogenid, gefälltes Hydroxid, Nitrat und Sulfat, in deionisiertem Wasser lösen, bis man eine klare Flüssigkeit erhält. Als vorteilhaft hat es sich erwiesen, dabei Aluminium in Form saurer Salze wie beispielsweise als Sulfat oder Nitrat einzusetzen. Bor setzt man vorzugsweise als Borsäure und Titan bevorzugt als Titanylsulfat ein.

Diese Aluminium-, Bor- und/oder Titanlösung kann man bevorzugt schnellstmöglich z.B. durch Zulaufen oder schnelles Zutropfen der auf Temperaturen von 20 bis 100°C, bevorzugt 40 bis 80°C und besonders bevorzugt 60 bis 75°C gehaltenen Suspension aus wäßrigem Alkylendi-, -tri- und/oder -tetramin und SiO₂-Quelle in den Druckrührbehälter geben. Zur besseren Durchmischung kann dabei die Rührgeschwindigkeit angehoben werden. Man erhält in der Regel ein Gel, das zur Homogenisierung für die Dauer von mehreren Stunden, in der Regel 0,2 bis 100 Stunden, bevorzugt 0,5 bis 20 Stunden, besonders bevorzugt 1 bis 6 Stunden auf den zuvorgenannten Temperaturen gehalten wird und heizt danach auf die zur Kristallisation benötigte Reaktionstemperatur von 110 bis 200°C, bevorzugt 130 bis 180°C, besonders bevorzugt 140 bis 160°C auf. Der Druck bei der Kristallisation beträgt in der Regel 0,01 bis 50 bar, bevorzugt 0,1 bis 5 bar, besonders bevorzugt arbeitet man unter Eigendruck. Die Bildung der Pentasilzeolithe tritt dabei in der Regel innerhalb von 1 bis 8 Tagen ein.

Zur Isolierung des Produktes kann man vom erkalteten Reaktionsaustrag den Feststoff durch Filtration abtrennen und den Filterkuchen mehrmals mit Wasser nachwaschen. Durch das erfindungsgemäße Verfahren fallen die Pentasilzeolithe in Form größerer hohlkugelartiger Agglomerate von 5 bis 200 micron, bevorzugt 10 bis 100 micron Partikelgröße (Durchmesser) an, so daß sich im Gegensatz zu den nach dem Stand der Technik herstellbaren Zeolithpulvern sehr kurze Filtrations- und Waschzeiten der Agglomerate realisieren lassen. Ebenso sinkt der mit diesem Arbeitsschritt häufig durch Trüblauf verbundene Produktverlust an Zeolith deutlich ab.

Anschließend kann der Feststoff bei 120°C getrocknet werden. Die hohlkugelartig agglomerierten Pentasilzeolithe in der Aminform können durch eine Temperaturbehandlung bei 400 bis 650°C, vorzugsweise 450 bis 550°C, besonders bevorzugt bei 480 bis 530°C in die katalytisch aktive H-Form des Zeolithen überführt werden.

Die erfindungsgemäßen hohlkugelartig agglomerierten Pentasilzeolithe haben in der Regel niedrige Schüttgewichte von 150 bis 280 kg/m³, bevorzugt 150 bis 200 kg/m³ mit verbesserter Fließ- und Wirbelfähigkeit.

Die erfindungsgemäßen Materialien lassen sich verfahrenstechnisch bevorzugt als Katalysatoren oder Adsorbentien z.B. in bewegten Schüttungen, Wirbelbetten und Suspensionen einsetzen.

Um eine möglichst hohe Selektivität, hohen Umsatz sowie lange Standzeiten als Katalysator zu erreichen, ist es vorteilhaft, die erfindungsgemäßen Zeolithe zu modifizieren. Eine geeignete Modifizierung besteht darin, daß man das unverformte oder verformte Material mit Metallsalzen durch einen Ionenaustausch oder durch Imprägnierung dotiert. Als Metalle werden Alkalimetalle wie Li, Cs, K, Erdalkalimetalle wie Mg, Ca, Sr, Metalle der 3., 4. und 5. Hauptgruppe wie Al, Ga, Ge, Sn, Pb, Bi, Übergangsmetalle der 4. bis 8. Nebengruppe wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe wie Cu, Ag, Zn, seltene Erdmetalle wie La, Ce, Pr, Nd, Fr, Yb und U eingesetzt.

Zweckmäßigerweise führt man die Dotierung so durch, daß man die erfindungsgemäßen Zeolithe in einem Steigrohr vorlegt und bei 20 bis 100°C eine wäßrige oder ammoniakalische Lösung eines Halogenids oder eines Nitrats der voranbeschriebenen Metalle über-leitet. Ein derartiger Ionenaustausch kann an der Wasserstoff-, Ammonium- und Alkaliform des Materials vorgenommen werden. Eine weitere Möglichkeit der Metallaufbringung auf die Hohlkugeln ist gegeben, indem man das erfindungs-gemäße Material z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man Cu(NO₃)₂ x 3 H₂O oder Ni(NO₃)₂ x 6 H₂O oder Ce(NO₃)₃ x 6 H₂O oder La(NO₃)₂ x 6 H₂O oder Cs₂CO₃ in Wasser löst. Mit dieser Lösung wird der agglomerierte Zeolith eine gewisse Zeit, etwa 30 Minuten, getränkt. Die eventuell überstehende Lösung wird am Rotationsverdampfer von Wasser befreit. Danach wird der getränkte Feststoff bei etwa 150°C getrocknet und bei etwa 550°C calciniert. Dieser Tränkvorgang kann mehrmals hintereinander vorgenommen werden, um den gewünschten Metallgehalt einzustellen.

Es ist möglich, eine wäßrige Ni(NO₃)₂-Lösung oder ammoniakalische Pd(NO₃)₂-Lösung herzustellen und darin die Hohlkugelagglomerate bei 40 bis 100°C unter Rühren etwa 24 h aufzuschlämmen. Nach Abfiltrieren, Trocknen bei etwa 150°C und Calcinierung bei etwa 500°C kann das so gewonnene Material dann gegebenenfalls noch mit oder ohne Bindemittel zu Strängen, Pellets oder Wirbeigut weiterverarbeitet werden. Wird bei dieser nachgeschalteten Verformung vorsichtig verfahren, so kann auf diesem Wege die Hohlkugelstruktur genutzt werden, um damit eine entsprechende Porosität im Bereich großer Transportporen (1 - 50 micron) dem Formgut aufzuprägen.

Ein Ionenaustausch des in der H-Form oder Ammonium-Form oder Alkali-Form vorliegenden Hohlkugel-Pentasilzeoliths kann so vorgenommen werden, daß man das Material in einer Kolonne vorlegt und darüber z.B. eine wäßrige Ni(NO₃)₂-Lösung oder ammoniakalische Pd(NO₃)₂-Lösung bei leicht erhöhter Temperatur zwischen 30 und 80°C im Kreislauf 15 bis 20 h leitet. Danach wird mit Wasser ausgewaschen, bei etwa 150°C getrocknet und bei etwa 550°C calciniert. Bei manchen metalldotierten erfindungsgemäßen Katalysatoren z.B. Pd-, Cu-, Ni- Dotierungen ist eine Nachbehandlung mit Wasserstoff vorteilhaft.

Eine weitere Möglichkeit der Modifizierung besteht darin, daß man das erfindungsgemäße Material - verformt oder unverformt - einer Behandlung mit Säuren wie Salzsäure, Flußsäure und Phosphorsäure und/oder Wasserdampf unterwirft. Dabei geht man vorteilhaft so vor, daß man den Zeolithen in Pulverform mit 1 n Phosphorsäure 1 Stunde bei 80°C behandelt. Nach der Behandlung wird mit Wasser gewaschen, bei 110°C/16 Stunden getrocknet und bei 500°C/20 Stunden calciniert.

Nach einer anderen Arbeitsweise behandelt man die Hohlkugeln vor oder nach ihrer Verformung mit Bindemitteln, z.B. 1 bis 3 Stunden bei Temperaturen von 60 bis 80°C mit einer 3 bis 25 gew.-%igen, insbesondere 12 bis 20 gew.-%igen wäßrigen Salzsäure. Anschließend wird der so behandelte Zeolith mit Wasser gewaschen, getrocknet und bei 400 bis 500°C calciniert.

Eine besondere Ausführungsform für die Säurebehandlung besteht darin, daß man das erfindungsgemäße Material vor seiner Verformung bei erhöhter Temperatur mit 0,001 n bis 2 n Flußsäure, yorzugsweise 0,05 n bis 0,5 n Flußsäure behandelt, beispielsweise durch Erhitzen unter Rückfluß über einen Zeitraum von 0,5 bis 5, vorzugsweise 1 bis 3 Stunden. Nach Isolierung, durch Abfiltrieren und Auswaschen, des erfindungsgemäßen Materials wird dieses zweckmäßig, bei Temperaturen von 100 bis 160°C, getrocknet und bei Temperaturen von 450 bis 600°C calciniert. Gemäß einer weiteren bevorzugten Ausführungsform für die Säurebehandlung wird das erfindungsgemäße Material nach einer Verformung mit Bindemittel bei erhöhter Temperatur, zweckmäßig bei 50 bis 90°C, vorzugsweise 60 bis 80°C, über einen Zeitraum von 0,5 bis 5, mit 12 bis 20 gew.-%iger Salzsäure, behandelt. Anschließend wird das erfindungsgemäße Material ausgewaschen und zweckmäßig, bei Temperaturen von 100 bis 160°C, getrocknet und bei Temperaturen von 450 bis 600°C calciniert. Einer HF-Behandlung kann sich auch eine HCl-Behandlung anschließen.

Die Umwandlungen an diesen Katalysatoren können in der Gasphase bei 100 bis 450°C, bevorzugt bei 150 bis 350°C, besonders bevorzugt bei 200 bis 300°C und einer Belastung WHSV = 0,1 bis 20 h⁻¹, bevorzugt 0,5 bis 5 h⁻¹ (g Ausgangsgemisch/g Katalysator und Stunde) in einem Festbett oder vorzugsweise auch im Wirbelbett ausgeführt werden.

Es ist bevorzugt möglich, die Reaktion in der Flüssigphase (Suspension-, Riesel- oder Sumpffahrweise) bei Temperaturen zwischen 0 und 250°C, bevorzugt zwischen 50 und 150°C durchzuführen.

Diese Verfahren werden in der Regel bei Drücken von 0,01 bis 50 bar, bevorzugt 0,1 bis 5 bar, besonders bevorzugt bei Normaldruck oder je nach Flüchtigkeit der Ausgangsverbindung bei vermindertem oder erhöhtem Druck durchgeführt, wobei die Durchführung vorzugsweise kontinuierlich, aber auch diskontinuierlich erfolgen kann.

Schwerflüchtige oder feste Ausgangsstoffe werden in gelöster Form z.B. in THF-, Toluol- oder Petrolether-Lösung zum Einsatz gebracht. Generell ist eine Verdünnung des Eduktes mit derartigen Lösungsmitteln oder mit Inertgasen wie N₂, Ar, H₂O-Dampf möglich.

Nach der Umsetzung können die entstandenen Produkte durch übliche Verfahren, z.B. durch Destillation, aus dem Reaktionsgemisch isoliert werden; nichtumgesetzte Ausgangsstoffe werden gegebenenfalls in die Umsetzung zurückgeführt. Aufgrund der erfindungsgemäßen Agglomerationsform des Zeolithen ist es ebenso möglich, den Katalysator durch eine einfache Filtration aus dem Reaktionsgemisch zurückzugewinnen.

Vorzugsweise können gasförmige Reaktionsprodukte sofort in eine Trennung eingebracht werden und z.B. in einer Fraktionierkolonne in ihre Einzelkomponenten zerlegt, um eine Rückreaktion zu unterbinden und um einen hohen Umsatz zu erzielen.

Die erfindungsgemäßen hohlkugelartig agglomerierten Pentasilzeolithe lassen sich als Heterogenkatalysatoren verwenden für nukleophile und elektrophile Substitutionen, für Addition- und Eliminierungsreaktionen, für Doppelbindungs- und Skelettisomerisierungen einschließlich Umlagerungen und z.B. für Alkylierungen, Isomerisierungen, Disproportionierungen, Acylierungen, Cyclisierungen, Hydratisierungen, Dehydratisierungen, Aminierungen, Hydrierungen, Dehydrierungen, Dehydrocyclisierungen, Hydroxylierungen, Skelettisomerisierungen sowie Kombinationen dieser Reaktionen, zur gezielten Umsetzung organischer Moleküle. Umsetzungen dieser Art sind beispielsweise in Hölderich, "Zeolites: Catalysis for the synthesis of organic compounds", Stud. Surf. Sci, Catal., Vol. 49, (1989) 69 bis 93 oder Hölderich und van Bekkum, "Zeolites in organic syntheses" Elsevier, Stud. Surf. Sci. Catal. Vol. 58 (1991) 631 bis 727 beschrieben. Besonders vorteilhaft lassen sich mit den erfindungsgemäßen Heterogenkatalysatoren Dehydratisierungsreaktionen ausführen.

Beispiele sind:
- Veresterungen, Carbonsäuren und Alkohole, z.B. Phthalsäureanhydrid und höherer Alkohol in der Flüssigphase
- Verätherungen aus Alkoholen
- Cyclisierungen von Diolen, z.B. 1,4-Butandiol zu THF.

Des weiteren lassen sich in Isomerisierungsreaktionen sowohl Doppelbindungs-als auch Skelettisomerisierungen mit den erfindungsgemäßen Pentasilzeolithen als Heterogenkatalysatoren durchführen.

Hierzu gehören:
- Epoxidumlagerung, d. h. Umwandlung eines Epoxides zu einem Aldehyd oder Keton, z.B. Styroloxid und seine Derivate zu den entsprechenden Phenylacetaldehyden.
- Beckmann-Umlagerung z.B. von Cyclohexanonoxim zu ε-Caprolactam.

Weiterhin lassen sich sowohl die sauer als auch bei entsprechend modifizierten Katalysatoren die basisch katalysierte Aldolkondensation mit den erfindungsgemäßen Katalysatoren durchführen. Beispiele sind:
- Aceton zu Mesityloxid
- Butyraldehyd zu 2-Ethylhexanal
- Acrolein, Formaldehyd und NH₃ zu Pyridin und β-Picolin.

Die erfindungsgemäßen Zeolithe können auch als Trägermaterialien für katalytisch aktive Komponenten wie Metalle durch Aufbringen über Imprägnierung oder Ionenaustausch wie zuvor erwähnt eingesetzt werden, z.B. für Edelmetalle in Hydrier- und Oxidationsreaktionen.

Neben den katalytischen Eigenschaften besitzen die Agglomerate auch Adsorptions- und Ionenaustauschkapazität und die erfindungsgemäßen Materialien können ebenso Verwendung finden als Adsorbentien für organische und anorganische Verbindungen.

### Beispiele

### Beispiel 1

Dieses Vergleichsbeispiel beschreibt die Kristallisation von Pentasilzeolithen ohne die erfindungsgemäße Agglomeration. In einem Druckrührbehälter wurden 14,2 kg einer wäßrigen Hexamethylendiaminlösung (50 Gew.-% in deionisiertem Wasser) und 13,5 kg deionisiertes Wasser vorgelegt. Unter Rühren setzt man dieser Mischung 4,6 kg Siliciumdioxid (Aerosil® 200, Fa. Degussa) hinzu und homogenisiert den Ansatz bei einer Rührgeschwindigkeit von 150 U/min. Aus 1,01 kg Aluminiumsulfat-18-hydrat (Fa. Merck) stellt man mit 6,76 kg Wasser eine klare Lösung her und fügt dies zum Reaktionsansatz hinzu. Das sich dabei bildende viskose Reaktionsgel wird bei 100 U/min homogenisiert und auf 150°C hochgeheizt. Die Kristallisation erfolgt bei 150°C für die Dauer von 168 Stunden. Das erkaltete Reaktionsgemisch wird aus dem Reaktor abgelassen und der gebildete Pentasilzeolith von der Mutterlauge abgetrennt.

Das Abfiltrieren der Mutterlauge und Neutralwaschen auf einer Druckfilternutsche (3 bar; 250 l Nenninhalt) dauerte 2-3 Stunden. Das anfangs trüb ablaufende Filtrat mußte zur Vermeidung von vermehrtem Produktverlust mehrfach über den Filterkuchen umgewälzt werden. Die Ausbeute an Zeolith auf eingesetztes Silica betrug 74 %. Das Schüttgewicht des getrockneten und bei 500°C für die Dauer von 12 Stunden kalzinierten Reaktionsproduktes beträgt 407 kg/m³. Die Größe der Primärkristallite, bestimmt aus rasterelektronenmikroskopischen Aufnahmen lag im Bereich von 0,2 bis 1 micron; bei dem kristallinen Produkt handelte es sich röntgenografisch um den in EP-A-7 081 beschriebenen Aluminiumsilikatzeolithen.

### Beispiel 2

In einem Druckrührbehälter wurden 14,2 kg einer wäßrigen Hexamethylendiaminlösung (50 Gew.-% in deionisiertem Wasser) und 13,4 kg deionisiertes Wasser vorgelegt. Unter Rühren setzt man dieser Mischung 4,6 kg Siliciumdioxid (Aerosil® 200, Fa. Degussa) innerhalb von 15 Minuten hinzu und homogenisiert den Ansatz bei einer Rührgeschwindigkeit von 150 U/min.

Aus 1,01 kg Aluminiumsulfat-18-hydrat (Fa. Merck) stellt man mit 6,76 kg Wasser eine klare Lösung her. Im Gegensatz zu Beispiel 1 heizt man den Reaktionsansatz zunächst auf 70°C hoch und fügt dann bei einer Rührerdrehzahl von 250 U/min die Aluminiumsulfatlösung schnell hinzu. Das gebildete Reaktionsgel wird bei 100 U/min für weitere vier Stunden homogenisiert und danach erst auf 150°C hochgeheizt. Die Kristallisation erfolgt bei 150°C für die Dauer von 168 Stunden. Das erkaltete Reaktionsgemisch wird aus dem Reaktor abgelassen und der gebildete Pentasilzeolith von der Mutterlauge abgetrennt. Das Abfiltrieren der Mutterlauge erforderte lediglich ca. 20 Minuten. Trüblauf des Filtrats wurde nicht festgestellt. Die Ausbeute an Zeolith auf eingesetztes Silica war quantitativ. Das Schüttgewicht des getrockneten und bei 500°C für die Dauer von 12 Stunden kalzinierten Reaktionsproduktes beträgt 173 kg/m³. Bei dem kristallinen Produkt handelte es sich röntgenografisch um den in EP-A-7 081 beschriebenen Aluminiumsilikat-Zeolithen.

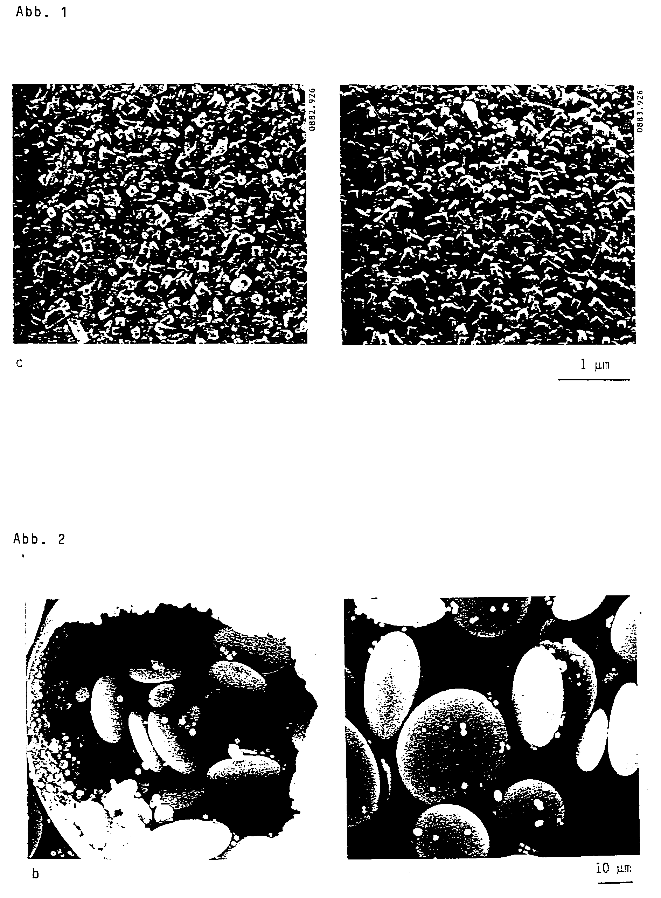

Aus der nachstehenden rasterelektronenmikroskopischen Abbildung 1 ist zu erkennen, daß es sich um verwachsene Kristallite primär einer Größe von etwa 0,1 bis 0,2 micron handelt. In Abbildung 2 ist zu sehen, daß diese Verwachsungen regelmäßig in Form von eiförmigen Hohlkugeln erfolgt sind, wobei die Größe der Hohlkugeln im Bereich von ca. 10 bis 100 micron liegt. Die Schalendicke solcher Hohlkugeln beträgt oft nur 2 bis 5 micron.
- Abbildung 1:: REM-Aufnahme der Primärkristallite
- Abbildung 2:: REM-Aufnahme der Hohlkugel-Agglomerate

## Patentansprüche

1. Hohlkugelartige agglomerierte Pentasilzeolithe mit einer Partikelgröße von 5 bis 200 micron.

2. Hohlkugelartige agglomerierte Pentasilzeolithe mit einer Partikelgröße von 10 bis 100 micron.

3. Hohlkugelartige agglomerierte Pentasilzeolithe mit einer Partikelgröße von 5 bis 200 micron hergestellt durch Behandlung einer Mischung aus einem wäßrigen Alkylendi-, -tri- und/oder -tetramin, Siliciumdioxid und einem Aluminium-, Bor- und/oder Titansalz bei Temperaturen von 20 bis 100°C, anschließende Kristallisation bei Temperaturen von 110 bis 200°C und Drücken von 0,01 bis 50 bar und Calcinierung bei Temperaturen von 400 bis 650°C.

4. Verfahren zur Herstellung von hohlkugelartig agglomerierten Pentasilzeolithen nach Anspruch 1, dadurch gekennzeichnet, daß man eine Mischung aus einem wäßrigen Alkylendi-, -tri- und/oder -tetramin, Siliciumdioxid und einem Aluminium-, Bor- und/oder Titansalz bei Temperaturen von 20 bis 100°C behandelt, anschließend bei Temperaturen von 110 bis 200°C und Drücken von 0,01 bis 50 bar kristallisiert und bei Temperaturen von 400 bis 650°C calciniert.

5. Verfahren zur Herstellung von hohlkugelartig agglomerierten Pentasilzeolithen nach Anspruch 3, dadurch gekennzeichnet, daß die Mischung ein wäßriges Alkylendi-, -tri- und/oder -tetramin, Siliciumdioxid, einem Aluminium- Bor- und/oder Titansalz im Wesentlichen keine Alkali- oder Erdalkaliverbindung enthält.

6. Verfahren zur Herstellung von hohlkugelartig agglomerierten Pentasilzeolithen nach Anspruch 3, dadurch gekennzeichnet, daß man eine wäßrige 20 bis 75%ige Lösung der Alkylendi-, -tri- und/oder -tetramine einsetzt.

7. Verfahren zur Herstellung von hohlkugelartig agglomerierten Pentasilzeolithen nach Anspruch 3, dadurch gekennzeichnet, daß man als Alkylendi-, -tri- und/oder -tetramin Triethylentetramin, Diethylentriamin, Dihexamethylentriamin, Dipropylentriamin, Propylendiamin, Dipropylentriamin, Dihexamethylentriamin, Triethylentetramin und/ oder Diethylentriamin einsetzt.

8. Verwendung der hohlkugelartig agglomerierten Pentasilzeolithe nach Anspruch 1 als Katalysatoren und/oder Adsorbentien.

## Claims

1. A pentasil zeolite agglomerated in the form of hollow spheres and having a particle size of from 5 to 200 micron.

2. A pentasil zeolite agglomerated in the form of hollow spheres and having a particle size of from 10 to 100 micron.

3. A pentasil zeolite agglomerated in the form of hollow spheres and having a particle size of from 5 to 200 micron, prepared by treating a mixture of an aqueous alkylenediamine, alkylenetriamine or alkylenetetramine, silica and an aluminum, boron or titanium salt at from 20 to 100°C, subsequent crystallization at from 110 to 200°C and from 0.01 to 50 bar and calcination at from 400 to 650°C.

4. A process for the preparation of a pentasil zeolite agglomerated in the form of hollow spheres, as claimed in claim 1, wherein a mixture of an aqueous alkylenediamine, alkylenetriamine or alkylenetetramine, silica and an aluminum, boron or titanium salt is treated at from 20 to 100°C and the product is then crystallized at from 110 to 200°C and from 0.01 to 50 bar and calcined at from 400 to 650°C.

5. A process for the preparation of a pentasil zeolite agglomerated in the form of hollow spheres, as claimed in claim 3, wherein the mixture contains an aqueous alkylenediamine, alkylenetriamine or alkylenetetramine, silica and an aluminum, boron or titanium salt and essentially no alkali metal compound or alkaline earth metal compound.

6. A process for the preparation of a pentasil zeolite agglomerated in the form of hollow spheres, as claimed in claim 3, wherein an aqueous 20-75% strength solution of the alkylenediamine, alkylenetriamine or alkylenetetramine is used.

7. A process for the preparation of a pentasil zeolite agglomerated in the form of hollow spheres, as claimed in claim 3, wherein triethylenetetramine, diethylenetriamine, dihexamethylenetriamine, dipropylenetriamine, propylenediamine, dipropylenetriamine, dihexamethylenetriamine, triethylenetetramine or diethylenetriamine is used as the alkylenediamine, alkylenetriamine or alkylenetetramine.

8. Use of a pentasil zeolite agglomerated in the form of hollow spheres, as claimed in claim 1, as a catalyst or adsorbent.

## Revendications

1. Zéolites du type pentasile sphériques creuses agglomérées, d'un calibre des particules de 5 à 200 microns.

2. Zéolites du type pentasile sphériques creuses agglomérées, d'un calibre des particules de 10 à 100 microns.

3. Zéolites du type pentasile sphériques creuses agglomérées, d'un calibre des particules de 5 à 200 microns, préparées par le traitement d'un mélange d'une alkylènedi-, -tri- et/ou -tétramine aqueuse, de dioxyde de silicium et d'un sel d'aluminium, de bore et/ou de titane, A des températures de 20 à 100°C, cristallisation subséquente à des températures de 110 à 200°C et sous des pressions de 0,01 à 50 bars et calcination à des températures de 400 à 650°C.

4. Procédé de préparation de zéolites du type pentasile sphériques creuses agglomérées suivant la revendication 1, caractérisé en ce que l'on traite un mélange d'une alkylènedi-, -tri- et/ou -tétramine aqueuse, de dioxyde de silicium et d'un sel d'aluminium, de bore et/ou de titane, à des températures de 20 à 100°C, puis on le cristallise à des températures de 110 à 200°C et sous des pressions de 0,01 à 50 bars et on le calcine à des températures de 400 à 650°C.

5. Procédé de préparation de zéolites du type pentasile sphériques creuses agglomérées suivant la revendication 3, caractérisé en ce que le mélange d'une alkylènedi-, -tri- et/ou -tétramine aqueuse, de dioxyde de silicium, d'un sel d'aluminium, de bore et/ou de titane ne contient sensiblement pas de composé de métal alcalin ou de métal alcalino-terreux.

6. Procédé de préparation de zéolites du type pentasile sphériques creuses agglomérées suivant la revendication 3, caractérisé en ce que l'on utilise une solution aqueuse à 20 à 75% de l'alkylènedi-, -tri- et/ou -tétramine.

7. Procédé de préparation de zéolites du type pentasile sphériques creuses agglomérées suivant la revendication 3, caractérisé en ce qu'à titre d'alkylènedi-, -tri- et/ou -tétramine, on utilise la triéthylènetétramine, la diéthylènetriamine, la dihexaméthylènetriamine, la dipropylènetriamine, la propylènediamine, la dipropylènetriamine, la dihexaméthylènetriamine, la triéthylènetétramine et/ou la diéthylènetriamine.

8. Utilisation de zéolites du type pentasile sphériques creuses agglomérées suivant la revendication 1, à titre de catalyseurs et/ou d'adsorbants.
